# EUROPÄISCHE PATENTSCHRIFT

(11) **EP 1 713 766 B1**
(45) Veröffentlichungstag und Bekanntmachung des Hinweises auf die Patenterteilung: **29.06.2011**
(21) Anmeldenummer: 05707007.0
(22) Anmeldetag: 26.01.2005
(51) Int. Cl.: C07C 253/34, C07C 255/07

(54) **VERFAHREN ZUR TRENNUNG VON PENTENNITRIL-ISOMEREN**
METHOD FOR THE SEPARATION OF PENTENENITRILE ISOMERS
PROCEDE DE SEPARATION D'ISOMERES PENTENENITRILE

(30) Priorität: 29.01.2004 DE 102004004721
(43) Veröffentlichungstag der Anmeldung: 25.10.2006
(73) Patentinhaber: BASF SE, 67056 Ludwigshafen (DE)
(72) Erfinder: JUNGKAMP, Tim, B-2950 Kapellen (BE); BAUMANN, Robert, 68159 Mannheim (DE); BARTSCH, Michael, 67433 Neustadt (DE); HADERLEIN, Gerd, 67269 Grünstadt (DE); LUYKEN, Hermann, 67069 Ludwigshafen (DE); SCHEIDEL, Jens, 69493 Hirschberg (DE)
(86) Internationale Anmeldenummer: PCT/EP2005/000726
(87) Internationale Veröffentlichungsnummer: WO 2005/073179

(56) Entgegenhaltungen:
- WO-A-02/26698
- US-A- 3 865 865

## Beschreibung

Die vorliegende Erfindung betrifft ein Verfahren zur Trennung von Stoffgemischen isomerer Pentennitrile.

Adipodinitril, ein wichtiges Intermediat in der Nylonproduktion, wird durch zweifache Hydrocyanierung von 1,3-Butadien hergestellt. Dabei wird in einer ersten Hydrocyanierung 1,3-Butadien mit Cyanwasserstoff in Gegenwart von Nickel(0), das mit Phosphorliganden stabilisiert ist, zu 3-Pentennitril umgesetzt. Nebenkomponenten dieser ersten Hydrocyanierung sind im Wesentlichen 2-Methyl-3-butennitril, 2-Pentennitrile, 2-Methyl-2-butennitrile, C₉-Nitrile, Methylglutarnitril und 4-Vinylcyclohexen. In einer zweiten Hydrocyanierung wird anschließend 3-Pentennitril mit Cyanwasserstoff zu Adipodinitril ebenfalls an einem Nickel-Katalysator, allerdings unter Zusatz einer Lewis-Säure umgesetzt. Auch in dieser zweiten Hydrocyanierung erhält man ein Gemisch aus den Edukt- und Produktnitrilen sowie den oben genannten Nebenkomponenten.

Die in diesen zwei Umsetzungen auftretenden komplexen Gemische müssen zur Durchführung eines wirtschaftlich attraktiven Verfahrens zur Herstellung von Adipodinitril voneinander getrennt werden. Aus den bekannten Verfahren zur Herstellung von Adipodinitril durch Hydrocyanierung von 1,3-Butadien und anschließender Umsetzung des hieraus resultierenden 3-Pentennitrils mit einem weiteren Molekül Cyanwasserstoff ist nicht bekannt, wie die komplexen Stoffgemische, insbesondere im Hinblick auf die Trennung von Pentennitril-Isomeren, aufgetrennt werden.

Wie in DE 100 49 265 beschrieben, bereitet die destillative Auftrennung von Pentennitrii-Isomeren erhebliche Probleme, da die relative Flüchtigkeit α der Pentennitril-Isomere bei Normaldruck im Bereich von 1,0 bis 2,0 und für eine Reihe von Isomerenpaare im Bereich von 1,0 bis 1,5 liegt. Unter der relativen Flüchtigkeit α versteht man dabei den Quotienten der Dampfdrücke zweier Substanzen, wobei man den Dampfdruck den Substanz mit dem höheren Dampfdruck in den Zähler des Quotienten nimmt.

| Pentennitril-Isomerenpaar | Relative Flüchtigkeit bei Normaldruck |
|---|---|
| 2-Methyl-3-butennitril / trans-3-Pentennitril | 1,72 |
| cis-2-Pentennitril / trans-3-Pentennitril | 1,55 |
| (E)-2-Methyl-2-butennitril / trans-3-Pentennitril | 1,19 |
| 2-Methyl-3-butennitril / (Z)-2-Methyl-2-butennitril | 1,12 |

Die destillative Trennung der genannten Spezies ist, da die relative Flüchtigkeit bei Normaldruck höher als 1,0 ist, zwar realisierbar, führt aber bei der Ausübung zu einem erheblichen technischen Aufwand und Energieverbrauch.

Zur Umgehung einer Trennung von trans-3-Pentennitril und trans-2-Pentennitril wird beispielsweise in US 3,526,654, US 3,564,040, US 3,852,325 und US 3,852,327 vorgeschlagen, die destillativ schlecht abtrennbaren Pentennitril-Isomere katalytisch in solche umzuwandeln, die sich leicht destillativ abtrennen lassen.

Nachteilig hierbei ist, dass die katalytische Isomerisierung durch Bildung von unerwünschten Isomeren oder Oligomeren zu Verlusten an Wertprodukten führt.

Zur Umgehung einer Trennung von (Z)-2-Methyl-2-butennitril und 2-Methyl-3-butennitril sowie trans-2-Pentennitril und trans-3-Pentennitril wird in US 3,865,865 vorgeschlagen, das Nitrilgemisch mit wässriger Sulfitlösung zu behandeln, jeweils unter Erhalt einer wässrigen Phase, enthaltend die jeweiligen Bisulfit-Addukte der konjugierten Nitrile (Z)-2-Methyl-2-butennitril bzw. trans-2-Pentennitril und einer an diesen Nitrilen abgereicherten organischen Phase. Nachteilig bei diesem Verfahren ist, dass die erhaltene organische Phase vor der Weiterverwendung in Hydrocyanierungsreaktionen unter Verwendung von Nickel(0)-Katalysatoren mit Phosphor(III)-haltigen. Liganden erst vollständig von Wasser befreit werden muss, da ansonsten die Phosphor(III)-haltigen Liganden irreversibel hydrolysiert und damit inaktiviert werden. Weiterhin nachteilig bei diesem Verfahren ist, dass die erhaltenen Bisulfit-Addukte zwecks Weiterverwendung der konjugierten Nitrile, wie in US 3,865,865 beschrieben, nur unter drastischen Bedingungen und nur mit mäßiger Ausbeute zurückspaltbar sind.

Zur Verbesserung der Trennbarkeit wird in DE 100 49 265 vorgeschlagen, durch Zusatz von flüssigen Verdünnungsmittel wie Wasser durch Extraktivdestillation die Abtrennbarkeit zu erhöhen. Auch dieses Verfahren hat den Nachteil, dass die erhaltenen Nitrilströme bei einer Weiterverarbeitung erst vom flüssigen Verdünnungsmittel, insbesondere Wasser, vollständig befreit werden müssen.

Der vorliegenden Erfindung liegt demnach die Aufgabe zugrunde, ein Verfahren bereitzustellen, das die destillative Auftrennung von Pentennitril-Isomeren, die bei Normaldruck eine relative Flüchtigkeit α im Bereich von 1,0 bis 2,0 aufweisen, auf technisch einfache und wirtschaftliche Weise ermöglicht.

Erfindungsgemäß wird die Aufgabe gelöst durch ein Verfahren zur Auftrennung von Stoffgemischen isomerer Pentennitrile, bei dem mindestens ein Isomer aus dem Gemisch abgereichert wird.

Das erfindungsgemäße Verfahren ist dadurch gekennzeichnet, dass die Auftrennung der Stoffgemische isomerer Pentennitrile destillativ unter vermindertem Druck erfolgt.

In dem erfindungsgemäßen Verfahren werden vorzugsweise mindestens zwei unterschiedliche Isomere aufgetrennt.

Das erfindungsgemäße Verfahren eignet sich vorzugsweise für Mischungen, die ausgewählt sind aus der Gruppe, bestehend aus
- Mischungen, enthaltend 2-Methyl-3-butennitril und 3-Pentennitril,
- Mischungen, enthaltend 2-Methyl-3-butennitril und (Z)-2-Methyl-2-butennitril,
- Mischungen, enthaltend cis-2-Pentennitril und 3-Pentennitril und
- Mischungen, enthaltend (E)-2-Methyl-2-butennitril und 3-Pentennitril.

Unter dem Begriff 3-Pentennitril werden im Sinne der vorliegenden Erfindung trans-3-Pentennitril oder Mischungen, die trans-3-Pentennitril und gegebenenfalls cis-3-Pentennitril und 4-Pentennitril enthalten, verstanden.

Die Hydrocyanierung von Butadien zu 3-Pentennitril und seinen Mischungen mit 2-Methyl-3-butennitril werden in Gegenwart eines Nickel(0)-Katalysators durchgeführt.

Bei den Ni(0)-Katalysatoren handelt es sich um Komplexe, die phosphorhaltige Liganden und/oder freie phosphorhaltige Liganden enthalten, bevorzugt um homogen gelöste Nickel(0)-Komplexe.

Die phosphorhaltigen Liganden der Nickel(0)-Komplexe und die freien phosphorhaltigen Ligandensind vorzugsweise ausgewählt aus mono- oder bidentaten Phosphinen, Phosphiten, Phosphiniten und Phosphoniten.

Diese phosphorhaltigen Liganden weisen vorzugsweise die Formel I auf:

P (X¹R¹) (X²R²) (X³R³) (I)

Unter Verbindung I wird im Sinne der vorliegenden Erfindung eine einzelne Verbindung oder ein Gemisch verschiedener Verbindungen der vorgenannten Formel verstanden. Erfindungsgemäß sind X¹, X², X³ unabhängig voneinander Sauerstoff oder Einzelbindung. Falls alle der Gruppen X¹, X² und X³ für Einzelbindungen stehen, so stellt Verbindung I ein Phosphin der Formel P(R¹R²R³) mit den für R¹, R² und R³ in dieser Beschreibung genannten Bedeutungen dar.

Falls zwei der Gruppen X¹, X² und X³ für Einzelbindungen stehen und eine für Sauerstoff, so stellt Verbindung I ein Phosphinit der Formel P(OR¹)(R²)(R³) oder P(R¹)(OR²)(R³) oder P(R¹)(R²)(OR³) mit den für R¹, R² und R³ weiter unten genannten Bedeutungen dar.

Falls eine der Gruppen X¹, X² und X³ für eine Einzelbindung steht und zwei für Sauerstoff, so stellt Verbindung I ein Phosphonit der Formel P(OR¹)(OR²)(R³) oder P(R¹)(CR²)(OR³) oder P(OR¹)(R²)(OR³) mit den für R¹, R² und R³ in dieser Beschreibung genannten Bedeutungen dar.

In einer bevorzugten Ausführungsform sollten alle der Gruppen X¹, X² und X³ für Sauerstoff stehen, so dass Verbindung I vorteilhaft ein Phosphit der Formel P(OR¹)(OR²)(OR³) mit den für R¹, R² und R³ weiter unten genannten Bedeutungen darstellt.

Erfindungsgemäß stehen R¹, R², R³ unabhängig voneinander für gleiche oder unterschiedliche organische Reste. Als R¹, R² und R³ kommen unabhängig voneinander Alkylreste, vorzugsweise mit 1 bis 10 Kohlenstoffatomen, wie Methyl; Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorzugsweise mit 1 bis 20 Kohlenstoffatomen, wie 1,1'-Biphenol, 1,1'-Binaphthol in Betracht. Die Gruppen R¹, R² und R³ können miteinander direkt, also nicht allein über das zentrale Phosphor-Atom, verbunden sein. Vorzugsweise sind die Gruppen R¹, R² und R³ nicht miteinander direkt verbunden.

In einer bevorzugten Ausführungsform kommen als Gruppen R¹, R² und R³ Reste ausgewählt aus der Gruppe bestehend aus Phenyl, o-Tolyl, m-Tolyl und p-Tolyl in Betracht. In einer besonders bevorzugten Ausführungsform sollten dabei maximal zwei der Gruppen R¹, R² und R³ Phenyl-Gruppen sein.

In einer anderen bevorzugten Ausführungsform sollten dabei maximal zwei der Gruppen R¹, R² und R³o-Tolyl-Gruppen sein.

Als besonders bevorzugte Verbindungen I können solche der Formel I a

(o-Tolyl-O-)_{w} (m-Tolyl-O-)ₓ (p-Tolyl-O-)_{y} (Phenyl-O-)_{z} P (Ia)

eingesetzt werden, wobei w, x, y und z eine natürliche Zahl bedeuten, und folgende Bedingungen gelten: w + x + y + z = 3 und w, z ≤ 2.

Solche Verbindungen I a sind z.B. (p-Tolyl-O-)(Phenyl-O-)₂P, (m-Tolyl-O-)(Phenyl-O-)₂P, (o-Tolyl-O-) (Phenyl-O-)₂P, (p-Tolyl-O-)₂(Phenyl-O-)P, (m-Tolyl-O-)₂(Phenyl-O-)P, (o-Tolyl-O-)₂(Phenyl-O-)P, (m-Tolyl-O-)(p-Tolyl-O)(Phenyl-O-)P, (o-Tolyl-O-)(p-Tolyl-O-)(Phenyl-O-)P, (o-Tolyl-O-)(m-Tolyl-O-)(Phenyl-O-)P, (p-Tolyl-O-)₃P, (m-Tolyl-O-)(p-Tolyl-O-)₂P, (o-Tolyl-O-)(p-Tolyl-O-)₂P, (m-Tolyl-O-)₂(p-Toluyl-O-)P, (o-Tolyl-O-)₂(p-Tolyl-O-)P, (o-Tolyl-O-)(m-Tolyl-O-)(p-Tolyl-O)P, (m-Tolyl-O-)₃P, (o-Tolyl-O-)(m-Tolyl-O-)₂P (o-Tolyl-O-)₂(m-Tolyl-O-)P, oder Gemische solcher Verbindungen.

Gemische enthaltend (m-Tolyl-O-)₃P, (m -Tolyl-O-)₂(p-Tolyl-O-)P, (m-Tolyl-O-)(p-Tolyl-O-)₂P und (p-Tolyl-O-)₃P kann man beispielsweise durch Umsetzung eines Gemisches enthaltend m-Kresol und p-Kresol, insbesondere im Molverhältnis 2 : 1, wie es bei der destillativen Aufarbeitung von Erdöl anfällt, mit einem Phosphortrihalogenid, wie Phosphortrichlorid, erhalten.

In einer anderen, ebenfalls bevorzugten Ausführungsform kommen als phosphorhaltige Liganden die in der DE-A 199 53 058 näher beschriebenen Phosphite der Formel I b in Betracht:

P (O-R)ₓ (O-R²)_{y} (O-R³)_{z} (O-R⁴)ₚ (Ib)

mit
- R¹:: aromatischer Rest mit einem C₁-C₁₈-Alkylsubstituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromati- schen System verbindet, anellierten aromatischen System,
- R²:: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoff- atom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der a- romatische Rest in o-Stellung zu dem Sauerstoffatom, das das Phosphoratom mit dem aromatischen System verbindet, ein Wasserstoffatom trägt,
- R³:: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aro- matische Rest in o-Stellung zu dem Sauerstoffatom, das das Phosphoratom mit dem aromatischen System verbindet, ein Wasserstoffatom trägt,
- R⁴:: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt,
- x :: 1 oder 2,
- y, z,: p: unabhängig voneinander 0, 1 oder 2 mit der Maßgabe, dass x+y+z+p = 3.

Bevorzugte Phosphite der Formel I b sind der DE-A 199 53 058 zu entnehmen. Als Rest R¹ kommen vorteilhaft o-Tolyl-, o-Ethyl-phenyl-, o-n-Propyl-phenyl-, o-Isopropyl-phenyl-, o-n-Butyl-phenyl-, o-sek-Butyl-phenyl-, o-tert-Butyl-phenyl-, (o-Phenyl)-Phenyl- oder 1-Naphthyl- Gruppen in Betracht.

Als Rest R² sind m-Tolyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen bevorzugt.

Als Rest R³ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Rest R⁴ ist bevorzugt Phenyl. Vorzugsweise ist p gleich null. Für die Indizes x, y, z und p in Verbindung I b ergeben sich folgende Möglichkeiten:

| x | y | z | p |
|---|---|---|---|
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

Bevorzugte Phosphite der Formel I b sind solche, in denen p gleich null ist sowie R¹, R² und R³ unabhängig voneinander ausgewählt sind aus o-Isopropyl-phenyl, m-Tolyl und p-Tolyl, und R⁴ Phenyl ist.

Besonders bevorzugte Phosphite der Formel I b sind solche, in denen R¹ der o-Isopropyl-phenyl-Rest, R² der m-Tolylrest und R³ der p-Tolylrest ist mit den in der vorstehenden Tabelle genannten Indizes; außerdem solche, in denen R¹ der o-Tolylrest, R² der m-Tolylrest und R³ der p-Tolylrest ist mit den in der Tabelle genannten Indizes; weiterhin solche, in denen R¹ der 1-Naphthylrest, R² der m-Tolylrest und R³ der p-Tolylrest ist mit den in der Tabelle genannten Indizes; außerdem solche, in denen R¹ der o-Tolylrest, R² der 2-Naphthylrest und R³ der p-Tolylrest ist mit den in der Tabelle genannten Indizes; und schließlich solche, in denen R¹ der o-Isopropyl-phenyl-Rest, R² der 2-Naphthylrest und R³ der p-Tolylrest ist mit den in der Tabelle genannten Indizes; sowie Gemische dieser Phosphite.

Phosphite der Formel I b können erhalten werden, indem man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹OH, R²OH, R³OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹OH, R²OH, R³OH und R⁴OH oder deren Gemische umsetzt unter Erhalt eines Phosphits der Formel I b.

Die Umsetzung kann in drei getrennten Schritten durchgeführt werden. Ebenso können zwei der drei Schritte kombiniert werden, also a) mit b) oder b) mit c). Alternativ können alle der Schritte a), b) und c) miteinander kombiniert werden.

Dabei kann man geeignete Parameter und Mengen der Alkohole ausgewählt aus der Gruppe bestehend aus R¹OH, R²OH, R³OH und R⁴OH oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere CI, eingesetzt wird, sowie deren Gemische in Betracht. Es können auch Gemische verschiedener gleich oder unterschiedlich halogensubstituierter Phosphine als Phosphortrihalogenid eingesetzt werden. Besonders bevorzugt ist PCl₃. Weitere Einzelheiten zu den Reaktionsbedingungen bei der Herstellung der Phosphite I b und zur Aufarbeitung sind der DE-A 199 53 058 zu entnehmen.

Die Phosphite I b können auch in Form eines Gemisches verschiedener Phosphite I b als Ligand verwendet werden. Ein solches Gemisch kann beispielsweise bei der Herstellung der Phosphite I b anfallen.

Es ist allerdings bevorzugt, dass der phosphorhaltige Ligand mehrzähnig, insbesondere zweizähnig ist. Daher weist der verwendete Ligand vorzugsweise die Formel II 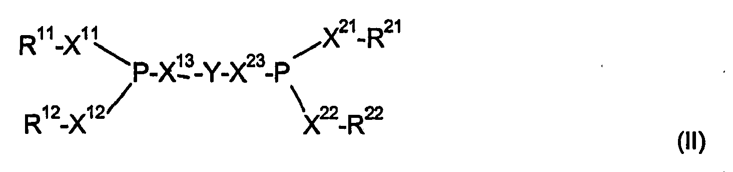 auf, worin bedeuten

| | |
|---|---|
| X¹¹, X¹², X¹³, X²¹, X²², X²³ | unabhängig voneinander Sauerstoff oder Einzelbindung |
| | |
| R¹¹, R¹² | unabhängig voneinander gleiche oder unterschiedliche, einzelne oder verbrückte organische Reste |
| | |
| R²¹, R²² Y | unabhängig voneinander gleiche oder unterschiedliche, einzelne oder verbrückte organische Reste, Brückengruppe |

Unter Verbindung II wird im Sinne der vorliegenden Erfindung eine einzelne Verbindung oder ein Gemisch verschiedener Verbindungen der vorgenannten Formel verstanden.

In einer bevorzugten Ausführungsform können X¹¹, X¹², X¹³, X²¹, X²², X²³ Sauerstoff darstellen. In einem solchen Fall ist die Brückengruppe Y mit Phosphit-Gruppen verknüpft.

In einer anderen bevorzugten Ausführungsform können X¹¹ und X¹² Sauerstoff und X¹³ eine Einzelbindung oder X¹¹ und X¹³ Sauerstoff und X¹² eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen bevorzugten Ausführungsform können X¹³ Sauerstoff und X¹¹ und X¹² eine Einzelbindung oder X¹¹ Sauerstoff und X¹² und X¹³ eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphinits oder Phosphins, vorzugsweise eines Phosphinits, sein kann.

In einer anderen bevorzugten Ausführungsform können X¹¹, X¹² und X¹³ eine Einzelbindung darstellen, so dass das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so dass das mit X²¹, X²² und X²³ umgebene Phosphoratom Zentralatom eines Phosphits oder Phosphins, vorzugsweise eines Phosphins, sein kann.

Als Brückengruppe Y kommen vorzugsweise substituierte, beispielsweise mit C₁-C₄-Alkyl, Halogen, wie Fluor, Chlor, Brom, halogeniertem Alkyl, wie Trifluormethyl, Aryl, wie Phenyl, oder unsubstituerte Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterscheidliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R¹¹ und R¹² können einzeln oder verbrückt sein. Auch die Reste R²¹ und R²² können einzeln oder verbrückt sein. Die Reste R¹¹, R¹², R²¹ und R²² können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht. In einer besonders bevorzugten Ausführungsform kommen die in US 5,512,696 genannten Verbindungen der Formel I, II, III IV, V, VI und VII, insbesondere die dort in den Beispielen 1 bis 31 eingesetzten Verbindungen, in Betracht. In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzten Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzten Verbindungen, in Betracht. In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzten Verbindungen, in Betracht.

In einer besonders bevorzugten Ausführungsform kommen die in US 6,127,567 genannten Verbindungen und dort in den Beispielen 1 bis 29 eingesetzten Verbindungen in Betracht. In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzten Verbindungen, in Betracht. In einer besonders bevorzugten Ausführungsform kommen die in US 5,959,135 genannten Verbindungen und dort in den Beispielen 1 bis 13 eingesetzten Verbindungen in Betracht.

In einer besonders bevorzugten Ausführungsform kommen die in US 5,847,191 genannten Verbindungen der Formel I, II und III in Betracht. In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 13, 14, 15, 16, 17, 18, 19, 20 und 21 dargestellten Verbindungen, in Betracht. In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht. In einer besonders bevorzugten Ausführungsform kommen die in WO 99/13983 genannten Verbindungen in Betracht. In einer besonders bevorzugten Ausführungsform kommen die in WO 99/64155 genannten Verbindungen in Betracht.

In einer besonders bevorzugten Ausführungsform kommen die in der deutschen Patentanmeldung DE 100 380 37 genannten Verbindungen in Betracht. In einer besonders bevorzugten Ausführungsform kommen die in der deutschen Patentanmeldung DE 100 460 25 genannten Verbindungen in Betracht. In einer besonders bevorzugten Ausführungsform kommen die in der deutschen Patentanmeldung DE 101 502 85 genannten Verbindungen in Betracht.

In einer besonders bevorzugten Ausführungsform kommen die in der deutschen Patentanmeldung DE 101 502 86 genannten Verbindungen in Betracht. In einer besonders bevorzugten Ausführungsform kommen die in der deutschen Patentanmeldung DE 102 071 65 genannten Verbindungen in Betracht. In einer weiteren besonders bevorzugten Ausführungsform der vorliegenden Erfindung kommen die in der US 2003/0100442 A1 genannten phosphorhaltigen Chelatliganden in Betracht.

In einer weiteren besonders bevorzugten Ausführungsform der vorliegenden Erfindung kommen die in der nicht vorveröffentlichten deutschen Patentanmeldung Aktenzeichen DE 103 50 999.2 vom 30.10.2003 genannten phosphorhaltigen Chelatliganden in Betracht.

Die beschriebenen Verbindungen I, I a, I b und II sowie deren Herstellung sind an sich bekannt. Als phosphorhaltiger Ligand können auch Mischungen, enthaltend mindestens zwei der Verbindungen I, I a, I b und II, eingesetzt werden.

In einer besonders bevorzugten Ausführungsform des erfindungsgemäßen Verfahrens ist der phosphorhaltige Ligand des Nickel(0)-Komplexes und/oder der freie phosphorhaltige Ligand ausgewählt aus Tritolylphosphit, bidentaten phosphorhaltigen Chelatliganden, sowie den Phosphiten der Formel I b

P(O-R¹)ₓ(O-R²)_{y}(O-R³)_{z}(O-R⁴)ₚ (I b)

worin R¹, R² und R³ unabhängig voneinander ausgewählt sind aus o-Isopropyl-phenyl, m-Tolyl und p-Tolyl, R⁴ Phenyl ist; x gleich 1 oder 2 ist, und y, z, p unabhängig voneinander 0, 1 oder 2 sind mit der Maßgabe, dass x+y+z+p = 3 ist; und deren Mischungen.

Die gewünschten Produkte dieser Hydrocyanierung, die zur Herstellung von Adipodinitril verwendet werden können, sind trans-3-Pentennitril, cis-3-Pentennitril und 4-Pentennitril, die im Sinne der vorliegenden Erfindung als 3-Pentennitril bezeichnet werden. Das in der Hydrocyanierung je nach Katalysatorsystem in mindestens zweistelligen Prozentmengen, bezogen auf die Summe aller gebildeten Pentennitrilisomere, anfallende 2-Methyl-3-butennitril muss vor der Weiterverarbeitung des Hydrocyanierungsaustrages allerdings abgetrennt werden. Die Spezifikation zur Abreicherung von 2-Methyl-3-butennitril in dem 3-Pentennitril ist scharf und muss eingehalten werden, weil in der nachfolgenden Hydrocyanierung aus 2-Methyl-3-butennitril Methylglutamitril (MGN), ein unerwünschtes Nebenprodukt der Adipodinitrilproduktion, entstehen würde. Der apparative Aufwand und der Energiebedarf zur nahezu vollständigen Abtrennung von 2-Methyl-3-butennitril von 3-Pentennitril ist jedoch sehr hoch und wird durch die relative Flüchtigkeit, definiert als Verhältnis der Dampfdrücke dieser beiden Stoffe, bestimmt, die bei Normaldruck ca. 1,7 beträgt, abgeleitet aus den bekannten Normalsiedepunkten von 416,8 K für trans-3-Pentennitril und 396,1 K für 2-Methyl-3-butennitril. Erfindungsgemäß wurde gefunden, dass die relative Flüchtigkeit von 2-Methyl-3-butennitril und 3-Pentennitril bei Drücken unterhalb des Normaldrucks ansteigt.

Demgemäß ist das erfindungsgemäße Verfahren in einer ***Ausführungsform I*** zur Auftrennung von Mischungen, die 2-Methyl-3-butennitril und 3-Pentennitril enthalten, bestimmt. Diese Mischungen werden vorzugsweise bei einer Umsetzung von 1,3-Butadien mit Cyanwasserstoff an einem Hydrocyanierungskatalysator erhalten, wobei der Hydrocyanierungsaustrag üblicherweise einen Anteil von in der Hydrocyanierung nicht umgesetztem 1,3-Butadien enthält, das durch geeignete Verfahren zumindest teilweise abgetrennt werden kann und wobei der in dem auf diese Weise erhaltenen Produktstrom enthaltene Katalysatoranteil durch geeignete Verfahren abgetrennt werden kann. Diesbezügliche Verfahren sind beispielsweise in der DE-A-102 004 004 720 (EP-A-1 716 104) und der DE-A-102 004 004 724 (EP-A-1 716 106) beschrieben. Eine Abtrennung des nicht umgesetzten 1,3-Butadiens und des Katalysatoranteils ist jedoch nicht zwingend erforderlich.

Die Mischung, die 2-Methyl-3-butennitril und 3-Pentennitril enthält, weist vorzugsweise einen Anteil an 2-Methyl-3-butennitril in der Mischung von 0,1 bis 99,9 Gew.-%, besonders bevorzugt 1 bis 99 Gew.%, insbesondere 10 bis 90 Gew.-%, jeweils bezogen auf die Summe der Pentennithlisomere in der Mischung, auf. Der Anteil an 3-Pentennitril in dieser Mischung beträgt vorzugsweise 0,1 bis 99,9 Gew.-%, besonders bevorzugt 1 bis 99 Gew.-%, insbesondere 10 bis 90 Gew.%, jeweils bezogen auf die Summe der Pentennitrilisomere in der Mischung.

Die Trennung der Mischung, die 2-Methyl-3-butennitril und 3-Pentennitril enthält, kann in jeder geeigneten, dem Fachmann bekannten Vorrichtung durchgeführt werden. Für die Destillation geeignet sind Apparaturen, wie sie beispielsweise in: Kirk-Othmer, Encyclopedia of Chemical Technology, 4.Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationsvorrichtungen sind jeweils mit geeigneten Vorrichtungen zur Verdampfung ausgerüstet, wie Fallfilmverdampfer, Dünnschichtverdampfer, Mehrphasenwendelrohrverdampfer, Naturumlaufverdampfer oder Zwangsumlaufentspannungsverdampfer sowie mit Vorrichtungen zur Kondensation des Brüdenstroms ausgerüstet. Die Destillation kann man in mehreren, wie 2 oder 3 Apparaturen, vorzugsweise in einer einzigen Apparatur durchführen. Die Destillation kann zudem einstufig im Sinne einer Teilverdampfung des Zulaufstroms erfolgen.

Die Anzahl der theoretischen Böden in der Destillationskolonne beträgt vorzugsweise 0 bis 100, besonders bevorzugt 1 bis 60, insbesondere 10 bis 40 Das Rücklaufverhältnis m(Kopfabzug) / m(Rücklauf auf Kolonne) beträgt vorzugsweise 0,01 bis 100, besonders bevorzugt 0,1 bis 10, insbesondere 0,2 bis 5, Der Zulauf in die Destillationskolonne kann flüssig oder gasförmig erfolgen. Der Zulauf in die Rektifikationskolonne kann in den Sumpf oder auf den Kopf der Kolonne erfolgen. Bevorzugt erfolgt der Zulauf auf die Höhe der Kolonne, die 1 bis 99 %, besonders bevorzugt 5 bis 90 %, insbesondere 10 bis 80 %, der Gesamtstufenzahl der Kolonne entspricht, jeweils vom Boden der Kolonne an gerechnet

Die Destillation der Mischung, enthaltend 2-Methyl-3-butennitril und 3-Pentennitril, erfolgt bei einem Druck von 0,02 bis 0,5 bar, insbesondere 0,05 bis 0,2 bar. Die Destillation wird vorzugsweise so durchgeführt, dass die Temperatur im Sumpf 20 bis 200 °C, besonders bevorzugt 30 bis 150 °C, insbesondere 50 bis 100 °C, beträgt. Die Destillation wird so durchgeführt, dass die Temperatur am Kopf vorzugsweise -15 bis 200 °C, besonders bevorzugt 0 bis 100 °C, insbesondere 20 bis 50 °C, beträgt. In einer besonders bevorzugten Ausführungsform des erfindungsgemäßen Verfahrens werden die zuvor genannten Temperaturbereiche sowohl im Sumpf, als auch am Kopf der Destillationsvorrichtung erreicht.

An der Destillationsvorrichtung wird am Kopf eine an 2-Methyl-3-butennitril gegenüber dem Zulaufstrom angereichte Mischung erhalten. Über Sumpf der Destillationsvorrichtung wird eine an 3-Pentennitril gegenüber dem Zulaufstrom angereicherte Mischung erhalten.

Das erfindungsgemäße Verfahren betrifft in einer ***Ausführungsform II*** die Auftrennung von Mischungen, die 2-Methyl-3-butennitril und (Z)-2-Methyl-2-butennitril enthalten.

Wie bereits oben beschrieben, wird in einem Hydrocyanierungsverfahren zur Herstellung von Adipodinitril zunächst 1,3-Butadien zu 3-Pentennitril hydrocyaniert. Dabei wird als Nebenprodukt 2-Methyl-3-butennitril erhalten. Dieses wird, wie in Ausführungsform I der vorliegenden Erfindung beschrieben, vorzugsweise vor einem zweiten Hydrocyanierungsschritt aus dem Reaktionsstrom abgetrennt. Das abgetrennte 2-Methyl-3-butennitril kann innerhalb eines integrierten Verfahrens zur Hydrocyanierung von 1,3-Butadien in zusätzlichen Verfahrensschritten zum Wertprodukt 3-Pentennitril isomerisiert werden. Während dieser Isomerisierung entsteht als Nebenprodukt (Z)-2-Methyl-2-butennitril, das von dem 2-Methyl-3-butennitril bei der Aufarbeitung des Isomerisierungsaustrags abgetrennt werden sollte, um Aufpegelungen im Verfahren bei Rückführung von 2-Methyl-3-butennitril in den Isomerisierungsschritt zu vermeiden.

(Z)-2-Methyl-2-butennitril ist von 2-Methyl-3-butennitril bei Normaldruck wegen nahezu gleicher Siedepunkte destillativ nicht mit wirtschaftlichem Aufwand trennbar. Der apparative Aufwand und der Energiebedarf zur Abreicherung von (Z)-2-Methyl-2-butennitril aus Mischungen, die 2-Methyl-3-butennitril enthalten, ist extrem hoch und wird durch die relative Flüchtigkeit, definiert als Verhältnis der Dampfdrücke dieser beiden Stoffe bestimmt, die bei Normaldruck ca. 1,1 beträgt, abgeleitet aus den bekannten Normalsiedepunkten von 392,1 K für (Z)-2-Methyl-3-butennitril und 396,1 K für 2-Mehtyl-3-butennitril. Erfindungsgemäß wurde gefunden, dass die relative Flüchtigkeiten von (Z)-2-Methyl-2-butennitril und 2-Methyl-3-butennitril bei Drücken unterhalb des Normaldrucks ansteigen, so dass eine Abtrennung von (Z)-2-Methyl-2-butennitril aus Mischungen, die 2-Methyl-3-butennitril enthalten, im Vakuum mit wirtschaftlichem Aufwand möglich ist.

Das erfindungsgemäße Verfahren ist somit vorzugsweise auch zur Trennung von Mischungen geeignet, die 2-Methyl-3-butennitril und (Z)-2-Methyl-2-butennitril enthalten, und beispielsweise aus einer Isomerisierung von 2-Methyl-3-butennitril stammen.

Die Isomerisierung wird vorzugsweise in Gegenwart eines Systems, enthaltend
a) Nickel(0),
b) eine Nickel(0) als Ligand komplexierende, dreibindigen Phosphor enthaltende Verbindung und
c) eine Lewis-Säure
   durchgeführt.

Die Herstellung der Nickel(0) enthaltenden Katalysatorsysteme kann nach an sich bekannten Verfahren erfolgen.

Als Liganden für den Isomerisierungskatalysator können die gleichen phosphorhaltigen Liganden wie für die oben beschriebene Hydrocyanierung verwendet werden.

Weiterhin kann das System eine Lewis-Säure enthalten.

Im Sinne der vorliegenden Erfindung wird unter einer Lewis-Säure eine einzelne Lewis-Säure, wie auch ein Gemisch aus mehreren, wie zwei, drei oder vier Lewis-Säuren, verstanden.

Als Lewis-Säure kommen dabei anorganische oder organische Metall-Verbindungen in Betracht, in denen das Kation ausgewählt ist aus der Gruppe bestehend aus Scandium, Titan, Vanadium, Chrom, Mangan, Eisen, Kobalt, Kupfer, Zink, Bor, Aluminium, Yttrium, Zirkonium, Niob, Molybdän, Cadmium, Rhenium und Zinn. Beispiele umfassen ZnBr₂, Znl₂, ZnCl₂, ZnSO₄, CuCl₂, CuCl, Cu(O₃SCF₃)₂, CoCl₂, Col₂, Fel₂, FeCl₃, FeCl₂, FeCl₂(THF)₂, TiCl₄(THF)₂, TiCl₄, TiCl₃, ClTi(O-i-Propyl)₃, MnCl₂, ScCl₃, AlCl₃, (C₈H₁₇)AlCl₂, (C₈H₁₇)₂AlCl, (i-C₄H₉)₂AlCl, (C₆H₅)₂AlCl, (C₆H₅)AlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆H₅)₃, TaCl₅, wie beispielsweise in US 6,127,567, US 6,171,996 und US 6,380,421 beschrieben. Weiterhin kommen in Betracht Metallsalze, wie ZnCl₂, Col₂ und SnCl₂ und organometallische Verbindungen, wie RAlCl₂, R₂AlCl, RSnO₃SCF₃ und R₃B, wobei R eine Alkyl- oder Aryl-Gruppe ist, wie beispielsweise in US 3,496,217, US 3,496,218 und US 4,774,353 beschrieben. Weiterhin können gemäß US 3,773,809 als Promotor ein Metall in kationischer Form, ausgewählt aus der Gruppe bestehend aus Zink, Cadmium, Beryllium, Aluminium, Gallium, Indium, Thallium, Titan, Zirkonium, Hafnium, Erbium, Germanium, Zinn, Vanadium, Niob, Scandium, Chrom, Molybdän, Wölfram, Mangan, Rhenium, Palladium, Thorium, Eisen und Kobalt, vorzugsweise Zink, Cadmium, Titan, Zinn, Chrom, Eisen und Kobalt, eingesetzt werden, wobei der anionische Teil der Verbindung ausgewählt sein kann aus der Gruppe bestehend aus Halogeniden, wie Fluorid, Chlorid, Bromid und Jodid, Anionen niedriger Fettsäuren mit von 2 bis 7 Kohlenstoffatomen, HPO₃²⁻, H₃PO²⁻, CF₃COO⁻, C₇H₁₅OSO₂⁻ oder SO₄²⁻. Weiterhin sind aus US 3,773,809 als geeignete Promotoren Borhydride, Organoborhydride und Borsäureester der Formel R₃B und B(OR)₃, wobei R ausgewählt ist aus der Gruppe bestehend aus Wasserstoff, Aryl-Radikale mit zwischen 6 und 18 Kohlenstoff-Atomen, mit Alkyl-Gruppen mit 1 bis 7 Kohlenstoff-Atomen substituierte Aryl-Radikale und mit Cyanosubstituierte Alkyl-Gruppen mit 1 bis 7 Kohlenstoff-Atomen substituierte Aryl-Radikale, vorteilhaft Triphenylbor, genannt. Weiterhin können, wie in US 4,874,884 beschrieben, synergistisch wirksame Kombinationen von Lewis-Säuren eingesetzt werden, um die Aktivität des Katalysatorsystems zu erhöhen. Geeignete Promotoren können beispielsweise aus der Gruppe bestehend aus CdCl₂, FeCl₂, ZnCl₂, B(C₆H₅)₃ und (C₆H₅)₃SnX, mit X=CF₃SO₃, CH₃C₆H₄SO₃ oder (C₆H₅)₃BCN ausgewählt werden, wobei für das Verhältnis von Promotor zu Nickel ein Bereich von vorzugsweise etwa 1:16 bis etwa 50:1 genannt ist.

Im Sinne der vorliegenden Erfindung umfasst der Begriff Lewis-Säure auch die in US 3,496,217, US 3,496,218, US 4,774,353, US 4,874,884, US 6,127,567, US 6,171,996 und US 6,380,421 genannten Promotoren.

Als besonders bevorzugte Lewis-Säuren kommen unter den genannten insbesondere Metallsalze, besonders bevorzugt Metallhalogenide, wie Fluoride, Chloride, Bromide, Jodide, insbesondere Chloride, in Betracht, von denen wiederum Zinkchlorid, Eisen-(II)-Chlorid und Eisen-(III)-chlorid besonders bevorzugt sind.

Die Isomerisierung kann in Gegenwart eines flüssigen Verdünnungsmittels, beispielsweise eines Kohlenwasserstoffs, wie Hexan, Heptan, Oktan, Cyclohexan, Methylcyclohexan, Benzol, beispielsweise eines Ethers, wie Diethylether, Tetrahydrofuran, Dioxan, Glykoldimethylether, Anisol, beispielsweise eines Esters, wie Ethylacetat, Methylbenzoat, oder beispielsweise eines Nitrils, wie Acetonitril, Benzonitril, oder Gemischen solcher Verdünnungsmittel durchgeführt werden. In einer besonders bevorzugten Ausführungsform kommt eine Isomerisierung in Abwesenheit eines solchen flüssigen Verdünnungsmittels in Betracht.

Weiterhin hat es sich als vorteilhaft erwiesen, wenn die Isomerisierung in nichtoxidierend wirkender Atmosphäre, wie beispielsweise unter einer Schutzgasatmosphäre aus Stickstoff oder einem Edelgas, wie Argon, durchgeführt wird.

Die Trennung der Mischung, die (Z)-2-Methyl-2-butennitril und 2-Methyl-3-Butennitril enthält, kann in jeder geeigneten, dem Fachmann bekannten Vorrichtung durchgeführt werden. Für die Destillation geeignet sind Apparaturen, wie sie beispielsweise in: Kirk-Othmer, Encyclopedia of Chemical Technology, 4.Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationsvorrichtungen sind jeweils mit geeigneten Vorrichtungen zur Verdampfung ausgerüstet, wie Fallfilmverdampfer, Dünnschichtverdampfer, Mehrphasenwendelrohrverdampfer, Naturumlaufverdampfer oder Zwangsumlaufentspannungsverdampfer sowie mit Vorrichtungen zur Kondensation des Brüdenstroms ausgerüstet. Die Destillation kann man in mehreren, wie 2 oder 3 Apparaturen, vorzugsweise in einer einzigen Apparatur durchführen. Die Destillation kann zudem einstufig im Sinne einer Teilverdampfung des Zulaufstroms erfolgen.

Die Anzahl der theoretischen Böden in der Destillationskolonne beträgt vorzugsweise 0 bis 100, besonders bevorzugt 1 bis 60, insbesondere 10 bis 40. Das Rücklaufverhältnis m(Kopfabzug) / m(Rücklauf auf Kolonne) beträgt vorzugsweise 0,1 bis 500, besonders bevorzugt 1 bis 200, insbesondere 10 bis 100. Der Zulauf in die Destillationskolonne kann flüssig oder gasförmig erfolgen. Der Zulauf kann auf die gesamte Höhe der Kolonne erfolgen, bevorzugt erfolgt der Zulauf auf die Höhe der Kolonne, die 0 bis 90 %, insbesondere 0 bis 50 %, der Gesamtstufenzahl der Kolonne entspricht, jeweils vom Boden der Kolonne an gerechnet.

Die in dem erfindungsgemäßen Verfahren gemäß Ausführungsform II verwendete Mischung weist vorzugsweise einen Anteil an 2-Methyl-3-butennitril in der Mischung von 0,1 bis 99 Gew.-%, besonders bevorzugt 1 bis 99 Gew.-%, insbesondere 10 bis 90 Gew.%, jeweils bezogen auf die Summe der Pentennitrilisomere in der Mischung, auf. Der Anteil an (Z)-2-Methyl-2-butennitril in dieser Mischung beträgt vorzugsweise 0,1 bis 99 Gew.-%, besonders bevorzugt 1 bis 90 Gew.%, insbesondere 2 bis 70 Gew.-%, jeweils bezogen auf die Summe der Pentennitrilisomere in der Mischung.

Das erfindungsgemäße Verfahren gemäß Ausführungsform II wird bei einem Druck von 0,02 bis 0,5 bar, insbesondere 0,05 bis 0,2 bar, durchgeführt. Die Destillation wird so durchgeführt, dass die Temperatur im Sumpf vorzugsweise 20 bis 200 °C, besonders bevorzugt 30 bis 150 °C, insbesondere 50 bis 100 °C, beträgt. Die Destillation wird so durchgeführt, dass die Temperatur am Kopf vorzugsweise -15 bis 200 °C, besonders bevorzugt 0 bis 100 °C, insbesondere 20 bis 50 °C, beträgt. In einer besonders bevorzugten Ausführungsform des erfindungsgemäßen Verfahrens werden die Temperaturbereiche sowohl im Sumpf, als auch am Kopf der Destillationskolonne eingehalten.

An der Destillationsvorrichtung wird am Kopf eine an 2-Methyl-3-butennitril gegenüber dem Zulaufstrom abgereichte Mischung erhalten. Über Sumpf der Destillationsvorrichtung wird eine an (Z)-2-Methyl-2-butennitril gegenüber dem Zulaufstrom abgereicherte Mischung erhalten.

Das erfindungsgemäße Verfahren betrifft in einer ***Ausführungsform III*** die Auftrennung von Mischungen, die cis-2-Pentennitril und 3-Pentennitril enthalten.

In der Hydrocyanierungsreaktion von 3-Pentennitril zu Adipodinitril entsteht cis-2-Pentennitril, das sich im 3-Pentennitril-Kreislaufstrom aufpegeln kann und somit vorzugsweise aus dem Kreislaufsystem entfernt wird.

Das abgetrennte cis-2-Pentennitril kann thermisch oder katalysiert zu dem Wertprodukt 3-Pentennitril isomerisiert werden. Dabei werden Mischungen mit im Wesentlichen trans-2-Pentennitril, trans-3-Pentennitril und nicht umgesetztem cis-2-Pentennitril erhalten. Voraussetzung für die Einbindung dieses Isomerisierungsschrittes in ein integrier tes Verfahren zur Herstellung von Adipodinitril ist, dass die Abtrennung von cis-2-Pentennitril aus dieser Mischung wirtschaftlich realisierbar ist.

Gemäß Ausführungsform III betrifft die vorliegende Erfindung somit ein Verfahren zur Trennung von Stoffgemischen isomerer Pentennitrile, wobei die Trennung destillativ im Vakuum erfolgt und die Mischung cis-2-Pentennitril und 3-Pentennitril enthält. Diese Mischungen stammen beispielsweise aus einer Umsetzung von 3-Pentennitril mit Cyanwasserstoff an einem Hydrocyanierungskatalysator. Darüber hinaus können diese Mischungen aus der thermischen oder katalysierten Isomerisierung von cis-2-Pentennitril stammen.

Der apparative Aufwand und der Energiebedarf zur Abreicherung von cis-2-Pentennitril von 3-Pentennitril ist hoch und wird durch die relative Flüchtigkeit α bestimmt, definiert als Verhältnis der Dampfdrücke dieser beiden Stoffe, die bei Normaldruck ca. 1,55 beträgt, abgeleitet aus den bekannten Normalsiedepunkten von 400,1 K für cis-2-Pentennitril und 416,8 K für trans-3-Pentennitril. Erfindungsgemäß wurde gefunden, dass die relative Flüchtigkeit von cis-2-Pentennitril und trans-3-Pentennitril bei Drücken unterhalb des Normaldrucks ansteigt.

Der Anteil an cis-2-Pentennitril in diesen Mischungen beträgt vorzugsweise 0,1 bis 99,9 Gew.%, besonders bevorzugt 1 bis 99 Gew.%, insbesondere 1 bis 90 Gew.-%, jeweils bezogen auf die Summe der Pentennitrilisomere in der Mischung. Der Anteil an 3-Pentennitril in der Mischung beträgt vorzugsweise 0,1 bis 99,9 Gew.-%, besonders bevorzugt 1 bis 99 Gew.%, insbesondere 2 bis 90 Gew.-%, jeweils bezogen auf die Summe der Pentennitrilisomere in der Mischung.

Die Trennung der Mischung, die cis-2-Pentennitril und 3-Pentennitril enthält, kann in jeder geeigneten, dem Fachmann bekannten Vorrichtung durchgeführt werden. Für die Destillation geeignet sind Apparaturen, wie sie beispielsweise in: Kirk-Othmer, Encyclopedia of Chemical Technology, 4.Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationsvorrichtungen sind jeweils mit geeigneten Vorrichtungen zur Verdampfung ausgerüstet, wie Fallfilmverdampfer, Dünnschichtverdampfer, Mehrphasenwendelrohrverdampfer, Naturumlaufverdampfer oder Zwangsumlaufentspannungsverdampfer sowie mit Vorrichtungen zur Kondensation des Brüdenstroms ausgerüstet. Die Destillation kann man in mehreren, wie 2 oder 3 Apparaturen, vorzugsweise in einer einzigen Apparatur durchführen. Die Destillation kann zudem einstufig im Sinne einer Teilverdampfung des Zulaufstroms erfolgen.

Die Anzahl der theoretischen Böden in der Destillationskolonne beträgt vorzugsweise 0 bis 100, besonders bevorzugt 1 bis 60, insbesondere 10 bis 40. Das Rücklaufverhältnis m(Kopfabzug) / m(Rücklauf auf Kolonne) beträgt vorzugsweise 0,1 bis 500, besonders bevorzugt 1 bis 200, insbesondere 10 bis 50. Der Zulauf in die Destillationskolonne kann flüssig oder gasförmig erfolgen. Der Zulauf kann auf die gesamte Höhe der Kolonne erfolgen, bevorzugt erfolgt der Zulauf auf die Höhe der Kolonne, die 0 bis 90 %, insbesondere 0 bis 50 %, der Gesamtstufenzahl der Kolonne entspricht, jeweils vom Boden der Kolonne an gerechnet

Die Trennung gemäß Ausführungsform III erfolgt bei einem Druck von 0,01 bis 0,5 bar, insbesondere 0,05 bis 0,2 bar. Die Destillation wird so durchgeführt, dass die Temperatur im Sumpf vorzugsweise 20 bis 200 °C, besonders bevorzugt 30 bis 150 °C, insbesondere 50 bis 100 °C, beträgt. Die Destillation wird so durchgeführt, dass die Temperatur am Kopf vorzugsweise -15 bis 200 °C, besonders bevorzugt 0 bis 100 °C, insbesondere 20 bis 50 °C, beträgt. In einer besonders bevorzugten Verfahrensweise des erfindungsgemäßen Verfahrens werden die Temperaturbereiche sowohl im Sumpf, als auch am Kopf der Kolonne eingehalten.

An der Destillationseinrichtung wird am Kopf eine an 3-Pentennitril gegenüber dem Zulaufstrom abgereicherte Mischung erhalten. Über Sumpf der Destillationseinrichtung wird eine an cis-2-Pentennitril gegenüber dem Zulaufstrom abgereicherte Mischung erhalten.

Das erfindungsgemäße Verfahren betrifft in einer ***Ausführungsform IV*** die Auftrennung von Mischungen, die 3-Pentennitril und (E)-2-Methyl-2-butennitril enthalten.

Während der Herstellung von 3-Pentennitril kann sowohl bei der Hydrocyanierung von 1,3-Butadien als auch bei der Isomerisierung von 2-Methyl-3-butennitril eine geringe Menge (E)-2-Methyl-2-butennitril als Nebenprodukt entstehen, wobei sich die gebildete Menge an (E)-2-Methyl-2-butennitril im Allgemeinen im Produktstrom zusammen mit 3-Pentennitril wiederfindet. Dieser Anteil an (E)-2-Methyl-2-butennitril pegelt sich in den bekannten Verfahren zur Hydrocyanierung von 3-Pentennitril zu Adipodinitril, wie in der DE-A-102 004 004 683 (EP-A-1 713 630) beschrieben, auf, da in der Hydrocyanierung die Anteile von nicht umgesetztem 3-Pentennitril zusammen mit (E)-2-Methyl-2-butennitril üblicherweise in die Reaktion zurückgeführt werden, wobei sich der Anteil von (E)-2-Methyl-2-buterinitril während der Reaktion im Wesentlichen inert verhält und nicht aus dem System entfernt werden kann, solange nicht zusätzliche Maßnahmen zur Ausschleusung des (E)-2-Methyl-2-butennitrils vorgesehen werden.

Der apparative Aufwand und der Energiebedarf zur Abreicherung von (E)-2-Methyl-2-butennitril aus 3-Pentennitril ist hoch und wird durch die relative Flüchtigkeit α bestimmt, definiert als Verhältnis der Dampfdrücke dieser beiden Stoffe, die bei Normaldruck ca. 1,19 beträgt, abgeleitet aus den bekannten Normalsiedepunkten von 410,1 K für (E)-2-Methyl-2-butennitril und 416,8 K für trans-3-Pentennitril. Erfindungsgemäß wurde gefunden, dass die relative Flüchtigkeit von (E)-2-Methyl-2-butennitril und 3-Pentennitril bei Drücken unterhalb des Normaldrucks ansteigt.

In den Hydrocyanierungsverfahren von 3-Pentennitril zu Adipodinitril pegelt sich (E)-2-Methyl-2-butennitril demnach gegebenenfalls im 3-Pentennitril-Kreislaufstrom auf. Dieser Kreislaufstrom kann sich als Mischung von Pentennitrilen beispielsweise einerseits aus dem Anteil an Pentennitrilen zusammensetzen, die direkt nach der Hydrocyanierungsreaktion abgetrennt werden. Andererseits können diesem Kreislaufstrom auch Pentennitrile zugeführt werden, die nach der Abtrennung von Katalysatorbestandteilen im Adipodinitril-haltigen Produktstrom verbleiben und aus diesem Strom zwecks Rückgewinnung abgetrennt werden, wie in der zeitgleich eingereichten DE-A-102 004 004 683 (EP-A-1 713 630) beschrieben.

Das erfindungsgemäße Verfahren ist gemäß der Ausführungsform IV demnach vorzugsweise für Mischungen bestimmt, die aus einer Umsetzung von 1,3-Butadien mit Cyanwasserstoff an einem Hydrocyanierungskatalysator oder aus einer Isomerisierung von 2-Methyl-3-butennitril oder aus einer Umsetzung von 3-Pentennitril mit Cyanwasserstoff an einem Hydrocyanierungskatalysator stammt.

In diesen Mischungen beträgt der Anteil an 3-Pentennitril vorzugsweise 0,1 bis 99,9 Gew.%, besonders bevorzugt 1 bis 99 Gew.%, insbesondere 10 bis 90 Gew.-%, jeweils bezogen auf die Summe der Pentennitrilisomere in der Mischung. Der Anteil an (E)-2-Methyl-2-butennitril in der Mischung beträgt vorzugsweise 0,1 bis 99,9 Gew.-%, besonders bevorzugt 0.5 bis 99 Gew.-%, insbesondere 1 bis 50 Gew.-%, jeweils bezogen auf die Summe der Pentennitrilisomere in der Mischung.

Die Trennung der Mischung, die 3-Pentennitril und (E)-2-Methyl-2-butennitril enthält, kann in jeder geeigneten, dem Fachmann bekannten Vorrichtung durchgeführt werden. Für die Destillation geeignet sind Apparaturen, wie sie beispielsweise in: Kirk-Othmer, Encyclopedia of Chemical Technology, 4.Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonne betrieben werden können. Diese Destillationsvorrichtungen sind jeweils mit geeigneten Vor richtungen zur Verdampfung ausgerüstet, wie Fallfilmverdampfer, Dünnschichtverdampfer, Mehrphasenwendelrohrverdampfer, Naturumlaufverdampfer oder Zwangsumlaufentspannungsverdampfer sowie mit Vorrichtungen zur Kondensation des Brüdenstroms ausgerüstet. Die Destillation kann man in mehreren, wie 2 oder 3 Apparaturen, vorzugsweise in einer einzigen Apparatur durchführen. Die Destillation kann zudem einstufig im Sinne einer Teilverdampfung des Zulaufstroms erfolgen.

Die Anzahl der theoretischen Böden in der Destillationskolonne beträgt vorzugsweise 0 bis 150, besonders bevorzugt 1 bis 120, insbesondere 10 bis 60. Das Rücklaufverhältnis m(Kopfabzug) / m(Rücklauf auf Kolonne) beträgt vorzugsweise 0,1 bis 500, besonders bevorzugt 1 bis 200, insbesondere 10 bis 100. Der Zulauf in die Destillationskolonne kann flüssig oder gasförmig erfolgen. Der Zulauf kann auf eine beliebige Stelle entlang der gesamten Höhe der Kolonne erfolgen, bevorzugt erfolgt der Zulauf auf die Höhe der Kolonne, die 0 bis 90 %, besonders bevorzugt 0 bis 50 %, der Gesamtstufenzahl der Kolonne entspricht, jeweils vom Boden der Kolonne an gerechnet

An der Destillationseinrichtung wird am Kopf eine an 3-Pentennitril gegenüber dem Zulaufstrom abgereichte Mischung erhalten. Über Sumpf der Destillationseinrichtung wird eine an (E)-2-Methyl-2-butennitril gegenüber dem Zulaufstrom abgereicherte Mischung erhalten.

Das erfindungsgemäße Verfahren der Trennung von Stoffgemischen isomerer Pentennitrile führt dazu, dass eine Trennung von 2-Methyl-3-butennitril beziehungsweise (E)-2-Methyl-2-butennitril mit jeweils 3-Pentennitril mit wenigen Trennstufen und geringer Energie erfolgen kann. Im Fall der Trennung von 2-Methyl-3-butennitril und (Z)-2-Methyl-2-butennitril sowie im Fall der Trennung von (E)-2-Methyl-2-butennitril und 3-Pentennitril ist die Trennung bzw. An- und Abreicherung überhaupt erst mit technisch realisierbarem Aufwand wirtschaftlich durchführbar.

Die vorliegende Erfindung wird anhand der vorliegenden Ausführungsbeispiele näher erläutert.

### Ausführungsbeispiele

Im Folgenden werden die folgenden Abkürzungen verwendet:

| | |
|---|---|
| T3PN | trans-3-Pentennitril |
| C3PN | cis-3-Pentennitril |
| 4PN | 4-Pentennitril |
| 2M3BN | 2-Methyl-3-butennitril |
| T2PN | trans-2-Pentenitril |
| C2PN | cis-2-Pentennitril |
| E2M2BN | (E)-2-Methyl-2-butennitril |
| Z2M2BN | (Z)-2-Methyl-2-butennitril |
| VSN | Valeriansäurenitril |
| VCH | 4-Vinylcyclohexen |

### 1. Mischungen, enthaltend 2-Methyl-3-butennitril und 3-Pentennitril

Die Trennung erfolgt in einer Destillationskolonne mit Verdampfer, Totalkondensator und Rücklaufteiler. Die Destillationskolonne umfasst 15 theoretische Böden. Das Rücklaufverhältnis m(Abzug) / m(Rücklauf auf Kolonne) beträgt 1. Der Zulauf beträgt 10 kg/h zu Stufe 10, gesehen von unten, auf die Destillationskolonne. Der Abzug am Kopf beträgt 5 kg/h.

Das Gemisch weist folgende Zusammensetzung auf:

**Tabelle 1**

| Bestandteil | Zulauf Gew.% |
|---|---|
| T3PN | 51 |
| C3PN | 1 |
| 4PN | 1 |
| 2M3BN | 34 |
| T2PN | 1 |
| C2PN | 1 |
| E2M2BN | 1 |
| Z2M2BN | 8 |
| VSN | 1 |
| VCH | 1 |

**Tabelle 2**

| Beispiel | Druck (bar) | Temperatur Sumpf °C | Temperatur Kopf °C | 2M3BN Sumpf Gew.-% | 3PN Kopf Gew.-% |
|---|---|---|---|---|---|
| 1 (Referenz) | 1.000 | 140 | 126 | 11 | 23 |
| 2 | 0.500 | 117 | 103 | 10 | 22 |
| 3 | 0.200 | 90 | 77 | 8 | 20 |
| 4 | 0.100 | 73 | 59 | 7 | 18 |
| 5 | 0.050 | 58 | 44 | 6 | 17 |
| 6 | 0.020 | 40 | 26 | 4 | 15 |

Die Beispiele 2 bis 6 zeigen, dass bei gleichem Rücklaufverhältnis und gleichen Abnahmemengen die Trennung von 2-Methyl-3-butennitril und 3-Pentennitril mit umso höherer Effizienz gelingt, je niedriger der Druck in der Kolonne unterhalb von 1.0 bar eingestellt wird: Bei niedrigerem Druck nimmt der Restgehalt von 2-Methyl-3-butennitril im Sumpf ab und der Restgehalt von trans-3-Pentennitril im Kopf nimmt auch ab.

### 2: Mischungen, enthaltend 2-Methyl-3-butennitril und (Z)-2-Methyl-2-butennitril

Die Trennung wird in einer Destillationskolonne mit Verdampfer, Totalkondensator und Rücklaufteiler durchgeführt. Die Destillationskolonne weist 15 theoretische Böden auf. Das Rücklaufverhältnis m(Abzug) / m(Rücklauf auf Kolonne) ist 50. Der Zulauf in den Verdampfer erfolgt mit 10 kg/h in den Sumpf der Kolonne, der Abzug am Kopf erfolgt mit 0,05 kg/h.

**Tabelle 3**

| Bestandteil | Zulauf Gew.-% |
|---|---|
| T3PN | 58 |
| C3PN | 1 |
| 4PN | 1 |
| 2M3BN | 21 |
| T2PN | 0 |
| C2PN | 1 |
| E2M2BN | 1 |
| Z2M2BN | 16 |
| VSN | 0 |
| VCH | 1 |

**Tabelle 4**

| Beispiel | Druck (bar) | Temperatur Sumpf °C | Temperatur Kopf °C | 2M3BN Kopf Gew.-% | Z2M2BN Kopf Gew.-% |
|---|---|---|---|---|---|
| 7 (Referenz) | 1.000 | 133 | 119 | 23 | 77 |
| 8 | 0.500 | 110 | 97 | 22 | 77 |
| 9 | 0.200 | 84 | 71 | 21 | 78 |
| 10 | 0.100 | 68 | 55 | 20 | 79 |
| 11 | 0.050 | 53 | 40 | 19 | 80 |
| 12 | 0.020 | 38 | 22 | 17 | 82 |

Die Beispiele 8 bis 12 zeigen, dass bei gleichem Rücklaufverhältnis und gleichen Abnahmemengen die Trennung von 2-Methyl-3-butennitril und (Z)-2-Methyl-2-butennitril mit umso höherer Effizienz gelingt, je niedriger der Druck in der Kolonne eingestellt wird: Bei niedrigerem Druck nimmt der Restgehalt von 2-Methyl-3-butennitril im Kopfabzug ab und der Gehalt vom abzutrennenden (Z)-2-Methyl-2-butennitril im Kopf nimmt zu.

### 3. Mischungen, enthaltend cis-2-Pentennitril und 3-Pentennitril

Die Trennung wird in einer Destillationskolonne mit Verdampfer, Totalkondensator und Rücklaufteiler durchgeführt. Die Destillationskolonne hat 15 theoretische Böden. Das Rücklaufverhältnis m(Abzug) / m(Rücklauf auf Kolonne) ist 50. Der Zulauf beträgt 10 kg/h auf Stufe 10, gesehen von unten, auf die Destillationskolonne. Der Abzug erfolgt am Kopf mit 0,05 kg/h.

**Tabelle 5**

| Bestandteil | Zulauf Gew.-% |
|---|---|
| T3PN | 68 |
| C3PN | 3 |
| 4PN | 5 |
| T2PN | 6 |
| C2PN | 9 |
| E2M2BN | 5 |
| Z2M2BN | 0 |
| VSN | 5 |
| VCH | 1 |

**Tabelle 6**

| Beispiel | Druck (bar) | Temperatur Sumpf °C | Temperatur Kopf °C | T3PN Kopf Gew.-% | C2PN Kopf Gew.-% |
|---|---|---|---|---|---|
| 13 | 0.02 | 46 | 29 | 7 | 86 |
| 14 | 0.05 | 61 | 47 | 10 | 82 |
| 15 | 0.1 | 75 | 63 | 13 | 79 |
| 16 | 0.2 | 92 | 80 | 16 | 75 |
| 17 | 0.5 | 118 | 108 | 20 | 69 |
| 18 (Referenz) | 1.00 | 141 | 132 | 24 | 63 |

Die Beispiele 13 bis 17 zeigen, dass bei gleichem Rücklaufverhältnis und gleichen Abnahmemengen die Trennung von trans-3-Pentennitril und cis-2-Pentennitril mit umso höherer Effizienz gelingt, je niedriger der Druck in der Kolonne eingestellt wird: Bei niedrigerem Druck nimmt der Restgehalt von trans-3-Pentennitril im Kopfabzug ab und der Gehalt vom abzutrennenden cis-2-Pentennitril im Kopf nimmt zu.

### 4. Mischung, enthaltend 3-Pentennitril und (E)-2-Methyl-2-butennitril

Die Trennung wird in einer Destillationskolonne mit Verdampfer, Totalkondensator und Rücklaufteiler durchgeführt. Die Destillationskolonne hat 40 theoretische Böden. Das Rücklaufverhältnis m(Abzug) / m(Rücklauf auf Kolonne) beträgt 50. Der Zulauf erfolgt mit 10 kg/h auf Stufe 5, gesehen von unten, auf die Destillationskolonne. Der Abzug am Kopf erfolgt mit 1,5 kg/h.

**Tabelle 7**

| Bestandteil | Zulauf Gew.-% |
|---|---|
| T3PN | 68 |
| C3PN | 3 |
| 4PN | 5 |
| T2PN | 6 |
| C2PN | 9 |
| E2M2BN | 5 |
| Z2M2BN | 0 |
| VSN | 5 |
| VCH | 1 |

**Tabelle 8**

| Beispiel | Druck (bar) | Temperatur Sumpf °C | Temperatur Kopf °C | E2M2NB Sumpf Gew.-% | E2M2BN Kopf Gew.-% |
|---|---|---|---|---|---|
| 19 | 0.02 | 55 | 31 | 1,4 | 25,5 |
| 20 | 0.05 | 67 | 49 | 1,5 | 24,7 |
| 21 | 0.1 | 80 | 65 | 1,7 | 24,0 |
| 22 | 0.2 | 95 | 82 | 1,8 | 23,3 |
| 23 | 0.5 | 121 | 109 | 1,9 | 22,6 |
| 24 (Referenz) | 1.00 | 144 | 132 | 1,9 | 22,4 |

Die Beispiele 19 bis 23 zeigen, dass bei gleichem Rücklaufverhältnis und gleichen Abnahmemengen die Trennung von trans-3-Pentennitril und (E)-2-Methyl-2-butennitril mit umso höherer Effizienz gelingt, je niedriger der Druck in der Kolonne eingestellt wird: Bei niedrigerem Druck nimmt der Restgehalt von (E)-2-Methyl-2-butennitril im Sumpfabzugsstrom ab und nimmt im Kopfabzugsstrom zu.

Aus Beispielen 1 bis 24 kann nach den allgemein bekannten Prinzipien der Destillation im Umkehrschluss abgeleitet werden, dass für das Erreichen einer geforderten Spezifikation der jeweiligen Pentennitrilisomere im Sumpf und Kopf der Destillationskolonne bei der Ausübung der Destillation im Vakuum weniger Trennstufen und / oder weniger Energie nötig ist, als aus der Betrachtung der bekannten Normalsiedepunkte zu erwarten wäre.

## Patentansprüche

1. Verfahren zur Auftrennung von Stoffgemischen isomerer Pentennitrile, bei dem mindestens ein Isomer aus dem Gemisch abgereichert wird, **dadurch gekennzeichnet, dass** die Trennung der Stoffgemische isomerer Pentennitrile ausgewählt sind aus der Gruppe bestehend aus
(i) - Mischungen, enthaltend 2-Methyl-3-butennitril und 3-Pentennitril,
(ii) - Mischungen, enthaltend 2-Methyl-3-butennitril und (Z)-2-Methyl-2-butennitril,
(iii)- Mischungen, enthaltend cis-2-Pentennitril und 3-Pentennitril und
(iv)- Mischungen, enthaltend (E)-2-Methyl-2-butennitril und 3-Pentennitril, wobei die Trennung der Stoffgemische (i), (ii) und (iv) destillativ unter einem Druck von 0,02 bis 0,5 bar erfolgt, und die Trennung des Stoffgemisches (iii) destilativ unter einem Druck von 0,01 bis 0,5 bar erfolgt.

2. Verfahren nach Anspruch 1, **dadurch gekennzeichnet, dass** mindestens zwei unterschiedliche Isomere aufgetrennt werden.

3. Verfahren nach einem der Ansprüche 1 oder 2, **dadurch gekennzeichnet, dass** die Mischung 2-Methyl-3-butennitril und 3-Pentennitril enthält und aus einer Umsetzung von 1,3-Butadien mit Cyanwasserstoff an einem Hydrocyanierungskatalysator stammt.

4. Verfahren nach Anspruch 3, **dadurch gekennzeichnet, dass** der Anteil an 2-Methyl-3-butennitril in der Mischung 0,1 bis 99,9 Gew.-%, bezogen auf die Summe aller Pentennitrilisomere in der Mischung, beträgt und/oder der Anteil an 3-Pentennitril in der Mischung 0,1 bis 99,9 Gew.-%, bezogen auf die Summe der Pentennitrilisomere in der Mischung, beträgt.

5. Verfahren nach einem der Ansprüche 1 oder 2, **dadurch gekennzeichnet, dass** die Mischung 2-Methyl-3-butennitril und (Z)-2-Methyl-2-butennitril enthält und aus einer Isomerisierung von 2-Methyl-3-butennitril stammt.

6. Verfahren nach Anspruch 5, **dadurch gekennzeichnet, dass** der Anteil an 2-Methyl-3-butennitril in der Mischung 0,1 bis 99 Gew.-%, bezogen auf die Summe der Pentennitrilisomere in der Mischung, beträgt und/oder der Anteil an (Z)-2-Methyl-2-butennitril in der Mischung 0,1 bis 99 Gew.-%, bezogen auf die Summe der Pentennitrilisomere in der Mischung, beträgt.

7. Verfahren nach einem der Ansprüche 1 oder 2, **dadurch gekennzeichnet, dass** die Mischung cis-2-Pentennitril und 3-Pentennitril enthält und aus einer Umsetzung von 3-Pentennitril mit Cyanwasserstoff an einem Hydrocyanierungskatalysator stammt

8. Verfahren nach Anspruch 7, **dadurch gekennzeichnet, dass** der Anteil an cis-2-Pentennitril in der Mischung 0,1 bis 99,9 Gew.-%, bezogen auf die Summe der Pentennitrilisomere in der Mischung, beträgt und/oder der Anteil an 3-Pentennitril in der Mischung 0,1 bis 99,9 Gew.-%, bezogen auf die Summe der Pentennitrilisomere in der Mischung, beträgt.

9. Verfahren nach einem der Ansprüche 1 oder 2, **dadurch gekennzeichnet, dass** die Mischung (E)-2-Methyl-2-butennitril und 3-Pentennitril enthält und aus einer Umsetzung von 1,3-Butadien mit Cyanwasserstoff an einem Hydrocyanierungskatalysator oder aus der Isomerisierung von 2-Methyl-3-butennitril oder aus einer Umsetzung von 3-Pentennitril mit Cyanwasserstoff an einem Hydrocyanierungskatalysator stammt

10. Verfahren nach Anspruch 9, **dadurch gekennzeichnet, dass** der Anteil an 3-Pentennitril in der Mischung 0,1 bis 99,9 Gew.-%, bezogen auf die Summe der Pentennitrilisomere in der Mischung, beträgt und/oder der Anteil an (E)-2-Methyl-2-butennitril in der Mischung 0,1 bis 99,9 Gew.-%, bezogen auf die Summe der Pentennitrilisomere in der Mischung, beträgt.

## Claims

1. A process for separating mixtures of isomeric pentenenitriles, in which at least one isomer is depleted from the mixture, wherein the mixtures of isomeric pentenenitriles are selected from the group consisting of
(i) - mixtures comprising 2-methyl-3-butenenitrile and 3-pentenenitrile,
(ii) - mixtures comprising 2-methyl-3-butenenitrile and (Z)-2-methyl-2-butenenitrile,
(iii) - mixtures comprising cis-2-pentenenitrile and 3-pentenenitrile and
(iv) - mixtures comprising (E)-2-methyl-2-butenenitrile and 3-pentenenitrile,
the mixtures (i), (ii) and (iv) being separated by distillation under a pressure of from 0.02 to 0.5 bar, and the mixture (iii) being separated by distillation under a pressure of from 0.01 to 0.5 bar.

2. The process according to claim 1, wherein at least two different isomers are separated.

3. The process according to either of claims 1 and 2, wherein the mixture comprises 2-methyl-3-butenenitrile and 3-pentenenitrile and stems from a reaction of 1,3-butadiene with hydrogen cyanide over a hydrocyanation catalyst.

4. The process according to claim 3, wherein the proportion of 2-methyl-3-butenenitrile in the mixture is from 0.1 to 99.9% by weight, based on the sum of all pentenenitrile isomers in the mixture, and/or the proportion of 3-pentenenitrile in the mixture is from 0.1 to 99.9% by weight, based on the sum of the pentenenitrile isomers in the mixture.

5. The process according to either of claims 1 and 2, wherein the mixture comprises 2-methyl-3-butenenitrile and (Z)-2-methyl-2-butenenitrile and stems from an isomerization of 2-methyl-3-butenenitrile.

6. The process according to claim 5, wherein the proportion of 2-methyl-3-butenenitrile in the mixture is from 0.1 to 99% by weight, based on the sum of the pentenenitrile isomers in the mixture, and/or the proportion of (Z)-2-methyl-2-butenenitrile in the mixture is from 0.1 to 99% by weight, based on the sum of the pentenenitrile isomers in the mixture.

7. The process according to either of claims 1 and 2, wherein the mixture comprises cis-2-pentenenitrile and 3-pentenenitrile and stems from a reaction of 3-pentenenitrile with hydrogen cyanide over a hydrocyanation catalyst.

8. The process according to claim 7, wherein the proportion of cis-2-pentenenitrile in the mixture is from 0.1 to 99.9% by weight, based on the sum of pentenenitrile isomers in the mixture, and/or the proportion of 3-pentenenitrile in the mixture is from 0.1 to 99.9% by weight, based on the sum of the pentenenitrile isomers in the mixture.

9. The process according to either of claims 1 and 2, wherein the mixture comprises (E)-2-methyl-2-butenenitrile and 3-pentenenitrile and stems from a reaction of 1,3-butadiene with hydrogen cyanide over a hydrocyanation catalyst or from the isomerization of 2-methyl-3-butenenitrile or from a reaction of 3-pentenenitrile with hydrogen cyanide over a hydrocyanation catalyst.

10. The process according to claim 9, wherein the proportion of 3-pentenenitrile in the mixture is from 0.1 to 99.9% by weight, based on the sum of the pentenenitrile isomers in the mixture, and/or the proportion of (E)-2-methyl-2-butenenitrile in the mixture is from 0.1 to 99.9% by weight, based on the sum of the pentenenitrile isomers in the mixture.

## Revendications

1. Procédé de séparation de mélanges de pentène-nitriles isomères, selon lequel au moins un isomère est extrait du mélange, **caractérisé en ce que** les mélanges de pentène-nitriles isomères sont choisis dans le groupe constitué par
(i) les mélanges contenant du 2-méthyl-3-butène-nitrile et du 3-pentène-nitrile,
(ii) les mélanges contenant du 2-méthyl-3-butène-nitrile et du (Z)-2-méthyl-2-butène-nitrile,
(iii) les mélanges contenant du cis-2-pentène-nitrile et du 3-pentène-nitrile, et
(iv) les mélanges contenant du (E)-2-méthyl-2-butène-nitrile et du 3-pentène-nitrile,
la séparation des mélanges (i), (ii) et (iv) ayant lieu par distillation à une pression de 0,02 à 0,5 bar et la séparation du mélange (iii) ayant lieu par distillation à une pression de 0,01 à 0,5 bar.

2. Procédé selon la revendication 1, **caractérisé en ce qu'**au moins deux isomères différents sont séparés.

3. Procédé selon l'une quelconque des revendications 1 ou 2, **caractérisé en ce que** le mélange contient du 2-méthyl-3-butène-nitrile et du 3-pentène-nitrile et est issu d'une réaction de 1,3-butadiène avec du cyanure d'hydrogène sur un catalyseur d'hydrocyanation.

4. Procédé selon la revendication 3, **caractérisé en ce que** la proportion de 2-méthyl-3-butène-nitrile dans le mélange est de 0,1 à 99,9 % en poids, par rapport à la somme de tous les isomères de pentène-nitrile dans le mélange, et/ou la proportion de 3-pentène-nitrile dans le mélange est de 0,1 à 99,9 % en poids, par rapport à la somme des isomères de pentène-nitrile dans le mélange.

5. Procédé selon l'une quelconque des revendications 1 ou 2, **caractérisé en ce que** le mélange contient du 2-méthyl-3-butène-nitrile et du (Z)-2-méthyl-2-butène-nitrile et est issu d'une isomérisation de 2-méthyl-3-butène-nitrile.

6. Procédé selon la revendication 5, **caractérisé en ce que** la proportion de 2-méthyl-3-butène-nitrile dans le mélange est de 0,1 à 99 % en poids, par rapport à la somme des isomères de pentène-nitrile dans le mélange, et/ou la proportion de (Z)-2-méthyl-2-butène-nitrile dans le mélange est de 0,1 à 99 % en poids, par rapport à la somme des isomères de pentène-nitrile dans le mélange.

7. Procédé selon l'une quelconque des revendications 1 ou 2, **caractérisé en ce que** le mélange contient du cis-2-pentène-nitrile et du 3-pentène-nitrile et est issu d'une réaction de 3-pentène-nitrile avec du cyanure d'hydrogène sur un catalyseur d'hydrocyanation.

8. Procédé selon la revendication 7, **caractérisé en ce que** la proportion de cis-2-pentène-nitrile dans le mélange est de 0,1 à 99,9 % en poids, par rapport à la somme des isomères de pentène-nitrile dans le mélange, et/ou la proportion de 3-pentène-nitrile dans le mélange est de 0,1 à 99,9 % en poids, par rapport à la somme des isomères de pentène-nitrile dans le mélange.

9. Procédé selon l'une quelconque des revendications 1 ou 2, **caractérisé en ce que** le mélange contient du (E)-2-méthyl-2-butène-nitrile et du 3-pentène-nitrile et est issu d'une réaction de 1,3-butadiène avec du cyanure d'hydrogène sur un catalyseur d'hydrocyanation ou de l'isomérisation de 2-méthyl-3-butène-nitrile ou d'une réaction de 3-pentène-nitrile avec du cyanure d'hydrogène sur un catalyseur d'hydrocyanation.

10. Procédé selon la revendication 9, **caractérisé en ce que** la proportion de 3-pentène-nitrile dans le mélange est de 0,1 à 99,9 % en poids, par rapport à la somme des isomères de pentène-nitrile dans le mélange, et/ou la proportion de (E)-2-méthyl-2-butène-nitrile dans le mélange est de 0,1 à 99,9 % en poids, par rapport à la somme des isomères de pentène-nitrile dans le mélange.
